# EUROPEAN PATENT APPLICATION

(11) **EP 3 239 292 A1**
(43) Date of publication of application: **01.11.2017**
(21) Application number: 15873195.0
(22) Date of filing: 24.12.2015
(51) Int. Cl.: C12N 5/00, A61K 35/42, A61P 9/12, A61P 11/00, C12N 5/02

(54) **CELL CULTURE SUPERNATANT FLUID DERIVED FROM LUNG TISSUE**

(30) Priority: 24.12.2014 JP 2014259827; 24.12.2014 JP 2014259829
(71) Applicant: Ube Industries, Ltd., Ube-shi, Yamaguchi 755-8633 (JP); National University Corporation Chiba University, Chiba, 2638522 (JP)
(72) Inventor: HAGIHARA, Masahiko, Ube-shi Yamaguchi 755-8633 (JP); KASUYA, Yoshitoshi, Chiba-shi Chiba 260-8670 (JP); TANAKA, Kensuke, Chiba-shi Chiba 260-8670 (JP); FUJITA, Tetsuo, Chiba-shi Chiba 260-8670 (JP)
(74) Representative: Ricker, Mathias
(86) International application number: PCT/JP2015/086063
(87) International publication number: WO 2016/104627

(57) **Abstract**

The purpose of the present invention is to provide a highly safe and highly effective pharmaceutical composition and the like for the prevention and treatment of respiratory diseases. The present invention provides a pharmaceutical composition with which it is possible to effectively repair damaged lung tissue by including a cell culture supernatant fluid derived from lung tissue. The present invention also provides a method and a kit for using said culture supernatant fluid to induce differentiation into lung surfactant protein positive cells.

## Description

### TECHNICAL FIELD

The present invention relates to a culture supernatant fluid of cells derived from lung tissue, a pharmaceutical composition containing this culture supernatant fluid for the treatment and/or prevention of respiratory diseases, and to a composition and method for inducing differentiation into pulmonary surfactant protein-positive cells using this culture supernatant fluid.

### BACKGROUND ART

A considerable increase in the incidence of respiratory diseases has been observed in recent years. Respiratory diseases is the generic term for diseases occurring in the so-called respiratory system (including the lungs, upper respiratory tract, trachea, bronchi and pleura) that present with symptoms associated with distress such as chest pain, coughing, breathing difficulty, hemoptysis or asthma, and occasionally may be serious to the extent of having an effect on vital prognosis. Although respiratory diseases can occur due to various causes such as infection, trauma or allergies, healing and repairing inflammation and damage to respiratory tissue is an important objective in terms of treatment.

However, there are many diseases for which the histological repair of lesions is difficult, such as in the case of pulmonary fibrosis, in which inflammatory tissue in the lungs undergoes fibrosis, or pulmonary edema or chronic obstructive pulmonary disease (COPD), in which alveoli are damaged. Although these diseases that exhibit an irreversible progression are treated to prevent further exacerbation using steroids, immunosuppressants or bronchodilators and the like (Patent Document 1), this treatment does not fundamentally improve the underlying condition.

The lungs are a constituent of the respiratory system and are mainly composed of the respiratory tract, blood vessels and alveoli. The role of the respiratory tract is to introduce oxygen in the air into the alveoli and expel carbon dioxide in the alveoli to the outside, and is composed of the upper respiratory tract (oral cavity, nasal cavity, pharynx and larynx) and lower respiratory tract (trachea, bronchi and bronchioles). Among these, the lower respiratory tract is present on the side of the respiratory tract closest to the lungs. The bronchi exhibit repeated branching as they move towards the periphery, resulting in the formation of bronchioles. The bronchioles undergo additional branching resulting in the formation of terminal bronchioles, which do not have a function that permits exchange of gas, and respiratory bronchioles, where exchange of gas takes place as a result of alveoli adhering to bronchiole walls. Respiratory bronchioles undergo several rounds of branching leading to the formation of alveolar ducts, alveolar sacs and alveoli. Alveoli account for 85% of lung volume and are organs that serve as sites for gas exchange with blood.

In addition to functioning as a physical barrier to foreign objects that have entered the lumen, luminal epithelium of the respiratory tract and alveoli also demonstrate functions such as maintaining a suitable level of humidity in the respiratory tract by secreting mucus or secreting various physiologically active substances such as pulmonary surfactant. Luminal epithelium is composed of various cells suited for these functions.

At sites closer to the trachea, the luminal epithelium (respiratory mucosal epithelium) exhibits a thick structure referred to as pseudostratified columnar ciliated epithelium in which basal cells, ciliated cells (columnar ciliated epithelial cells) and mucus-secreting cells (goblet cells and Clara cells) are arranged on the basal lamina. However, as the respiratory tract become progressively branched beyond the trachea, the structure of the luminal epithelium changes. At the terminal bronchioles, the luminal epithelium exhibits a simple cuboidal epithelium structure, which is replaced with epithelium composed of squamous type I alveolar epithelial cells when the luminal epithelium reaches the respiratory bronchioles. Epithelial cells present in the respiratory bronchiole region, excluding type I alveolar epithelial cells, are referred to as small airway epithelial cells (SAEC). Alveolar epithelium is composed of type I alveolar epithelial cells and type II alveolar epithelial cells. Among these, type II alveolar epithelial cells are known to produce pulmonary surfactant, which is a substance that reduces alveolar surface tension, as well as various inflammatory cytokines. In recent years, type II alveolar epithelial cells have been suggested to have the potential to function as the initial targets of TGF-β or have the potential to function as pulmonary stem/progenitor cells (PSPC) during the course of pulmonary fibrosis, and are recognized to be important lung constituent cells associated with lung diseases (Non-Patent Documents 1 and 2). The aforementioned pulmonary surfactant is useful in reducing alveolar surface tension and preventing alveolar collapse (Non-Patent Document 3). In diseases in which type I alveolar epithelial cells are damaged, type II alveolar epithelial cells and their surrounding cells are known to proliferate and subsequently differentiate into type I alveolar epithelial cells to fulfill the role of repairing tissue (Non-Patent Documents 4 and 5). However, the direct administration of type II alveolar epithelial cells for the purpose of prevention or treatment of respiratory diseases, including lung injury, had problems from the viewpoint of histocompatibility, such as the risk of the occurrence of host-versus-graft rejection. Thus, there is a need for a more effective and safer method for the prevention and treatment of respiratory diseases, including lung injury.

Although commonly known, various lung diseases can occur when cells that compose the lungs become damaged or inflamed. There are many lung diseases for which histological repair of lesions is difficult, such as bronchiolitis obliterans (BO), which presents with respiratory failure caused by irreversible obstruction of the bronchioles, or chronic obstructive pulmonary disease (COPD), in which alveoli are damaged. Although these irreversibly progressive diseases are treated by preventing further exacerbation through the use of steroids, immunosuppressants or bronchodilators and the like (Patent Document 2), they fail to bring about fundamental improvement of the underlying condition.

As has been previously described, pulmonary surfactant protein-positive cells in the form of type II alveolar epithelial cells and small airway epithelial cells play an important role in the onset and progression of irreversibly progressive lung diseases such as pulmonary fibrosis, bronchiolitis obliterans or chronic obstructive pulmonary disease. For example, in diseases associated with damage to type I alveolar epithelial cells, type II alveolar epithelial cells are known to differentiate into type I alveolar epithelial cells and subsequently proliferate to fulfill the role of tissue repair. In addition, the repair of damaged small airway epithelial cells is important for the improvement and healing of bronchial asthma and bronchiolitis. Moreover, determining the manner in which pulmonary surfactant protein-positive cells such as type II alveolar epithelial cells or small airway epithelial cells are able to differentiate is expected to lead to elucidation of the repair mechanism of these pulmonary surfactant protein-positive cells as well as an understanding of the pathology and the development of treatment methods for irreversibly progressive lung diseases. In order to accomplish this, there is a desire for the development of technology capable of inducing differentiation from cells not destined to differentiate into the pulmonary surfactant protein-positive cells into pulmonary surfactant protein-positive cells.

Previous known methods for obtaining pulmonary surfactant protein-positive cells consisted of recovering cells already present in lung tissue. For example, a known method for obtaining type II alveolar epithelial cells consisted of enzymatically dissociating lung tissue cells followed by selectively isolating the cells using antibody specific to type II alveolar epithelial cells and recovering those cells (Non-Patent Document 3). In addition, a known method for obtaining small airway epithelial cells consists of scraping cells directly from bronchioles with a brush followed by isolating and recovering the cells (Non-Patent Document 6).

However, there is currently no known method capable of inducing differentiation from cells not destined to differentiate into pulmonary surfactant protein-positive cells into pulmonary surfactant protein-positive cells.

### [Prior Art Documents]

### [Patent Documents]

[Patent Document 1] WO2006/043655
[Patent Document 2] JP2013-544235A

### [Non-Patent Documents]

[Non-Patent Document 1] Journal Clinical Investigation (2011) 121, 277-287
[Non-Patent Document 2] Journal Clinical Investigation (2013) 123, 3025-3036
[Non-Patent Document 3] Laboratory Investigation (2004) 84, 727-735
[Non-Patent Document 4] Laboratory Investigation (2014) 94, 1247-1259
[Non-Patent Document 5] American Journal Respiratory Critical Care Medicine (2007) 176, 1261-1268
[Non-Patent Document 6] Respiratory Research (2009) 10: 99

### DISCLOSURE OF THE INVENTION

### [Problems to be Solved by the Invention]

An object of the present invention is to provide a pharmaceutical composition for the prevention and/or treatment of respiratory diseases that contains a cell culture supernatant fluid derived from lung tissue that is highly safe and highly effective. Moreover, another object of the present invention is to provide a composition, kit and method for inducing differentiation from cells not destined to differentiate into pulmonary surfactant protein-positive cells into pulmonary surfactant protein-positive cells using this culture supernatant fluid.

### [Means for Solving the Problems]

As a result of having subjected cells isolated from lung tissue to mixed culturing, the inventors of the present invention previously found that type II alveolar epithelial cell-like cells can be obtained in the form of pulmonary surfactant protein-positive cells (Non-Patent Document 4). It was also found that lung injury is inhibited by transpulmonary administration of a culture supernatant fluid to lung injury model mice at the time type II alveolar epithelial cell-like cells are obtained. Moreover, it was also found that cell culture supernatant fluid obtained by culturing these cells has the ability to induce various cells such as peripheral blood mononuclear cells not destined to differentiate into pulmonary surfactant protein-positive cells to differentiate into pulmonary surfactant protein-positive cells, thereby leading to completion of the present invention.

The present invention provided the following.
[1] A culture supernatant fluid of cells derived from lung tissue.
[2] The culture supernatant fluid described in [1], wherein the cells derived from lung tissue are spherical cells expressing one or more proteins, or mRNA encoding the same, selected from the group consisting of pulmonary surfactant protein or a precursor thereof, CD44 and CD45.
[3] The culture supernatant fluid described in [2], wherein the pulmonary surfactant protein is pulmonary surfactant protein C (SP-C).
[4] The pharmaceutical composition described in [2] or [3], wherein the expression level of one or more of the proteins, or mRNA encoding the same, selected from the group consisting of CD34 and CD90 is 5% or less of the expression level of all proteins or all mRNA.
[5] A method for preparing a culture supernatant fluid of cells derived from lung tissue, comprising:
   (1) a step for forming a cell suspension by suspending cells isolated from lung tissue in a medium,
   (2) a step for culturing the cell suspension followed by removing non-adherent cells,
   (3) a step for further culturing the remaining cells in a medium to form spherical cells, and
   (4) a step for recovering the cell culture
   supernatant fluid.
[6] A pharmaceutical composition for the treatment and/or prevention of respiratory diseases, containing the culture supernatant fluid described in any of [1] to [4] or the culture supernatant fluid prepared by the method described in [5].
[7] The pharmaceutical composition described in [6], wherein the respiratory disease is a lung disease or bronchial asthma.
[8] The pharmaceutical composition described in [7], wherein the lung disease is selected from the group consisting of chronic obstructive pulmonary disease (COPD), pulmonary fibrosis, acute respiratory distress syndrome, pulmonary hypertension, pneumonia and pulmonary sarcoidosis.
[9] A method for producing pulmonary surfactant protein-positive cells, comprising: contacting the culture supernatant fluid described in any of [1] to [4] or a culture supernatant fluid prepared according to the method described in [5] with cells selected from the group consisting of peripheral blood mononuclear cells, mesenchymal stem cells and embryonic stem cells (ES cells).
[10] A kit for producing pulmonary surfactant protein-positive cells, including the culture supernatant fluid described in any of [1] to [4] or a culture supernatant fluid prepared according to the method described in [5].

### [Effects of the Invention]

Use of the cell culture supernatant fluid derived from lung tissue of the present invention or a pharmaceutical composition containing that cell culture supernatant fluid makes it possible to effectively repair damaged lung tissue. In addition, the pharmaceutical composition of the present invention is highly safe from the viewpoint of histocompatibility since it does not contain cell bodies or tissue fragments. In this manner, the cell culture supernatant fluid derived from lung tissue of the present invention or a pharmaceutical composition containing that cell culture supernatant fluid can be expected to be used to treat and prevent respiratory diseases.

As a result of contacting a cell culture supernatant fluid derived from lung tissue with various cells, including somatic cells, such as peripheral blood mononuclear cells, and stem cells, such as ES cells, the present invention is able to induce differentiation from these cells into pulmonary surfactant protein-positive cells. In addition, the present invention is able to induce differentiation from cells not destined to differentiate into pulmonary surfactant protein-positive cells into pulmonary surfactant protein-positive cells.

In addition to greatly contributing to regenerative medicine for the treatment and/or prevention of lung disease and other respiratory diseases, the cell culture supernatant fluid derived from lung tissue, pharmaceutical composition and method of the present invention are useful in research on diseases associated with pulmonary surfactant protein-positive cells and the development of therapeutic drugs thereof. Moreover, since the pulmonary surfactant protein-positive cells obtained according to the present invention can be produced using autologous cells, they demonstrate high compatibility with autologous tissue and are suited for use in transplant therapy. In addition, since the pulmonary surfactant protein-positive cells obtained according to the present invention can be produced using peripheral blood mononuclear cells or other blood-derived cells, they can be produced in large volume while being lowly invasive to the body.

### BRIEF DESCRIPTION OF THE DRAWINGS

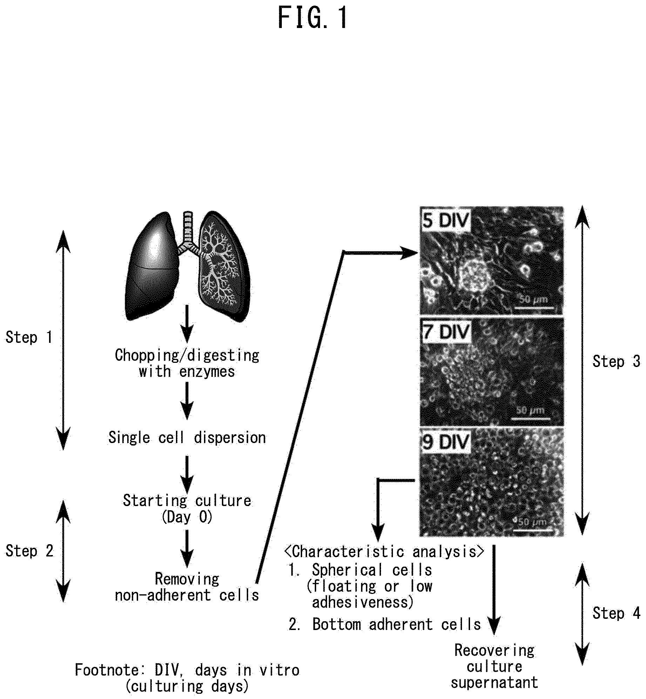

[Figure 1] FIG. 1 indicates a process for isolating lung mixed culture-derived epithelial cells (LMDEC) (type II alveolar epithelial cell-like cells) and recovering culture supernatant fluid. The drawing indicates time-dependent changes in lung-derived mixed culturing. Sphere-like cell aggregates appeared on day 5 of culturing, diffused on day 7 of culturing, and a large number of cells that floated or were loosely adhered on adherent cells were observed on day 9 of culturing.

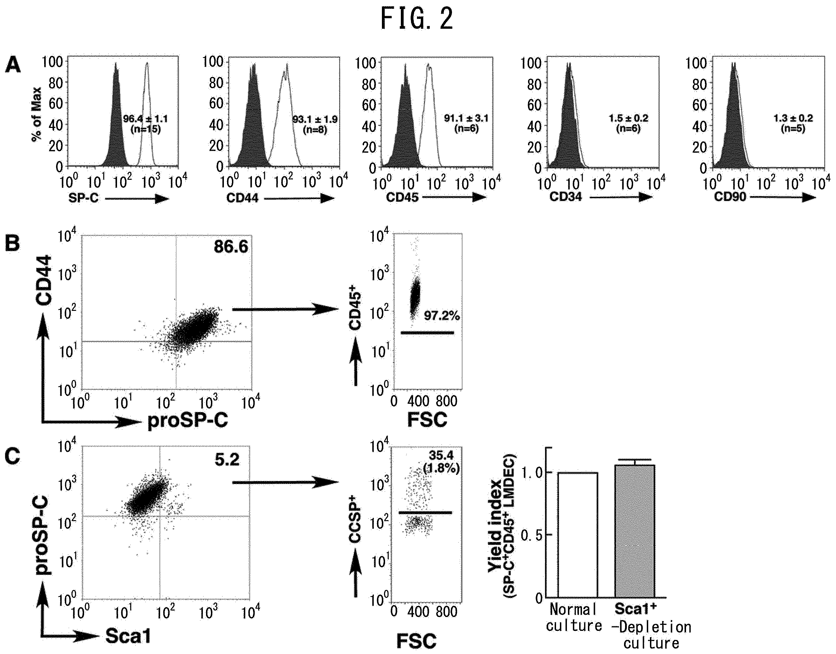

[Figure 2] FIG. 2 indicates the results of flow cytometry analysis of LMDEC. The histograms respectively indicate (A) the percentages of proSP-C-positive, CD44-positive, CD45-positive, CD34-positive and CD90-positive LMDEC. The shaded histograms indicate LMDEC used as a negative control that were stained with a suitable isotype control or secondary antibody. (B) The majority of the SP-C-positive LMDEC co-expressed CD44 and CD45. FSC is the abbreviation for forward scatter. (C) Small groups of LMDEC are SP-C-positive/Sca1-positive/CCSP-positive and suggest BASC. Depletion of Sca1-positive cells containing BASC did not affect the yield of SP-C-positive/CD45-positive LMDEC. The yield of the SP-C-positive/CD45-positive LMDEC from the group in which Sca1-positive cells had been depleted is expressed as a ratio with the cell count of SP-C-positive/CD45-positive LMDEC cells from a normal group. The shaded bar is expressed as the mean ± standard error (n=3). There were no significant differences between the two groups.

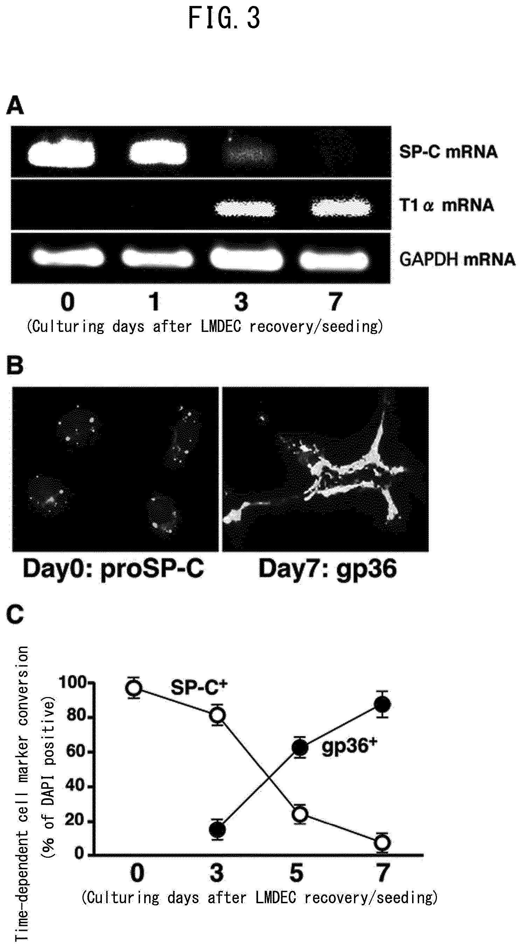

[Figure 3] FIG. 3 indicates the differentiation potency characteristics of LMDEC into type I alveolar epithelial cells. (A) This drawing indicates time-dependent changes in SP-C and T1α mRNA. Total RNA of spherical cells was prepared at the times indicated and subjected to PCR for the purpose of amplifying mouse SP-C or T1α. (B) Although LMDEC were SP-C-positive immediately after recovering from a mixed culture, they were gp36-positive on day 7 of culturing. (C) This graph indicates the inverse correlation between the percentages of SP-C-positive LMDEC and gp36-positive LMDEC on the basis of culture time.

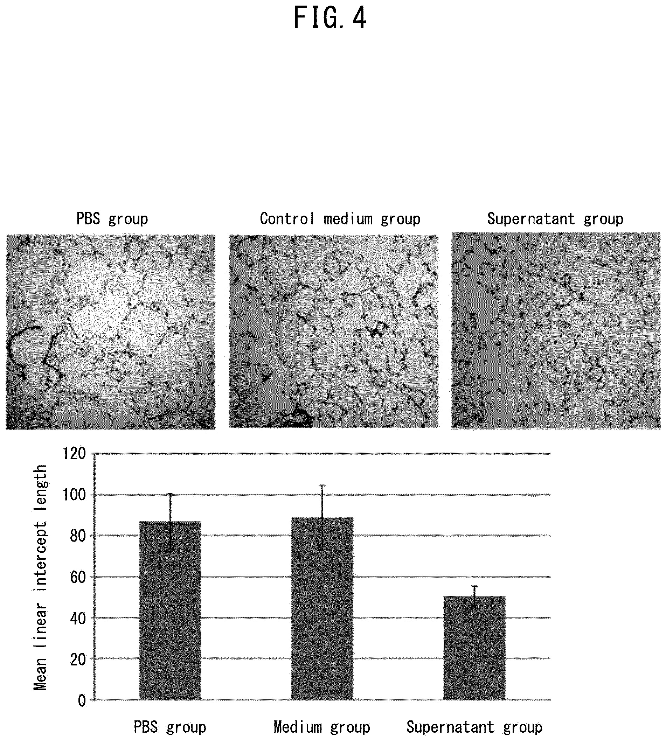

[Figure 4] FIG. 4 indicates the results of tissue analyses of mouse lung administered with a culture supernatant fluid of cells derived from lung tissue, control medium or PBS following elastase treatment. In the upper images, (1) indicates the PBS dose group, (2) indicates the control medium (10% FBS/DMEM) group, and (3) indicates an image obtained by tissue staining mouse lung from the group administered lung tissue-derived cell mixed culture supernatant fluid. The lower graph indicates the results of graphing the mean linear intercept length for each group (1) to (3).

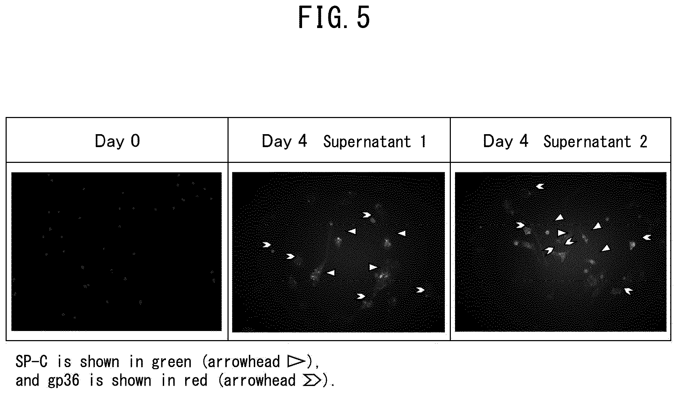

[Figure 5] FIG. 5 indicates the results of cell induction using mouse LMDEC culture supernatant fluid. SP-C is shown in green (arrowhead), while gp36 is shown in red (arrowhead).

### MODE FOR CARRYING OUT THE INVENTION

According to the present invention, a pharmaceutical composition for the treatment and prevention of respiratory diseases, and a culture supernatant fluid of cells derived from lung tissue for use in a composition and method for producing pulmonary surfactant protein-positive cells, are provided.

### A. Culture Supernatant Fluid of Cells Derived from Lung Tissue

In the present invention, "lung tissue cells" refer to cells that compose lung tissue. Cells that compose lung tissue are cells that compose the respiratory tract (lower respiratory tract), blood vessels and alveoli contained in the lungs.

The lower respiratory tract is composed of the trachea, bronchi and bronchioles. The lower respiratory tract is composed of respiratory mucosal epithelium, basal lamina, lamina propria mucosae, cartilage and an outer membrane composed of fat tissue moving in order from the lumen. At sites closer to the trachea, the respiratory mucosal epithelium exhibits a thick structure referred to as pseudostratified columnar ciliated epithelium in which basal cells, ciliated cells (columnar ciliated epithelial cells) and mucus-secreting cells (goblet cells and Clara cells) are arranged on the basal lamina. However, as the respiratory tract become progressively branched beyond the trachea, the structure of the luminal epithelium changes. At the terminal bronchioles, the luminal epithelium exhibits a simple cuboidal epithelium structure, which is replaced with epithelium composed of squamous type I alveolar epithelial cells when the luminal epithelium reaches the respiratory bronchioles. Epithelial cells present in the respiratory bronchiole region, excluding type I alveolar epithelial cells, are referred to as small airway epithelial cells (SAEC).

Alveoli are composed of alveolar space that stores gas, and alveolar epithelium that surrounds that space. The alveolar epithelium is composed of type I alveolar epithelial cells and type II alveolar epithelial cells. Type I alveolar epithelial cells are extremely thin, squamous cells referred to as respiratory epithelial cells or squamous alveolar epithelial cells. Adjacent areas of cytoplasm adhere by tight junctions and form blood-air barriers by adhering with capillary endothelial cells via the basal lamina. Gas exchange takes place at this site between oxygen in the alveoli and carbon dioxide in the blood. Type II alveolar epithelial cells are also referred to as large alveolar epithelial cells or granular alveolar epithelial cells. These cells have microvilli present on the cell surface, and secretory granules rich in phospholipid are present in the cytoplasm. These cells secrete a surfactant in the form of pulmonary surfactant by exocytosis that reduces the surface tension of alveoli, resulting in the formation of the alveolar lining layer. Other examples of cells that form lung tissue include vascular endothelial cells, alveolar septal cells, fibroblasts and macrophages present in alveoli and interstitium.

In the present invention, "lung-tissue derived cells" refer to cells obtained from lung tissue.

Examples of methods used to obtain culture supernatant fluid of lung-tissue derived cells used in the present invention include, but are not limited to, the method indicated below.
(1) A cell suspension is formed by suspending isolated cells obtained from mammalian lung tissue in a medium.
(2) The cell suspension is cultured followed by removing non-adherent cells.
(3) The remaining cells are further cultured in a medium to form spherical cells.
(4) The cell culture supernatant fluid is recovered.

In step (1) of the aforementioned method, lung derived from, for example, a human, dog, cat, cow, horse, domestic rabbit, hamster, guinea pig, pig, monkey, mouse or rat can be preferably applied as the mammalian lung.

The lung obtained from a mammal has preferably been removed of blood.

Any method may be used to obtain isolated cells from the lung. For example, the lung can be mechanically cut into thin sections with a scissors and the like followed by digesting by treating with enzyme. Any enzyme may be used for enzymatic treatment provided it is an enzyme that is able to digest connective tissue and mutually dissociate the cells. For example, deoxyribonuclease (DNase), protease such as trypsin or dispase, collagenase or a mixture of one or more types thereof can be used. Examples of DNase include, but are not limited to, DNase I and DNase II. Examples of proteases include, but are not limited to, serine proteases such as trypsin, chymotrypsin or subtilin, aspartic proteases such as pepsin or cathepsin D, and cysteine proteases such as papain, actinidin, ficin, cathepsin B, cathepsin K, cathepsin L, cathepsin S or caspase. Examples of collagenases include, but are not limited to, type I collagenase, type II collagenase, type III collagenase, type IV collagenase, type V collagenase, type VI collagenase, type VII collagenase and type VIII collagenase.

Any medium may be used to suspend the resulting isolated lung cells provided it is a medium that allows culturing of mammalian cells. Examples of media that can be used preferably include MEM medium, DMEM medium, Ham's F12 medium, Eagle's MEM medium, RPMI1640 medium and mixed media thereof. Serum such as fetal bovine serum (FBS), newborn bovine serum (NBS), calf serum (CS) or equine serum (ES) can be added as necessary to the medium within a range of 5% by weight to 30% by weight and preferably 10% by weight to 20% by weight. In addition, a serum-free medium such as PMPro, OptiPro or SFM may also be used. In mice, the isolated lung cells are preferably suspended in medium at 1 × 10⁶ cells/ml of medium to 1 × 10⁸ cells/ml of medium and more preferably suspended at 1 × 10⁷ cells/ml of medium to 2 × 10⁷ cells/ml of medium. In humans, although the cells can be suspended at 1 × 10⁶ cells/ml of medium to 1 × 10⁸ cells/ml of medium, they may also be suspended at 1 × 10⁷ cells/ml of medium to 2 × 10⁷ cells/ml of medium.

In step (2) of the aforementioned method, any conditions may be used for the culturing conditions provided they allow culturing of mammalian cells. For example, conditions consisting of 5% CO₂ and 37°C can be used. Although the duration of culturing can be suitably determined by a person with ordinary skill in the art corresponding to the species of animal, it is preferably 1 day to 5 days and more preferably 2 days to 3 days. After culturing, non-adherent cells are removed by a method such as aspiration or decantation, fresh medium is added after washing at least twice using a buffer solution, and the cells are further washed at least once with medium. 1 × PBS, for example, can be preferably used for the buffer solution used during washing.

In step (3) of the aforementioned method, the remaining cells are further cultured. The same conditions as those used in step (2) may be used for the culturing conditions. Although the duration of culturing can be suitably determined by a person with ordinary skill in the art corresponding to the species of animal, it is preferably 3 days to 90 days and more preferably 7 days to 60 days. This step results in the formation of spherical cells. Spherical cells refer to cells having a roughly spherical shape measuring 1 µm to 30 µm in diameter, and can be confirmed with a microscope.

In the previously described preparation method, following step (3), the spherical cells can be confirmed to be type II alveolar epithelial cells-like cells (used with the same meaning as "pulmonary surfactant protein-positive cells" in the present description) by confirming the expression of one or more proteins, or mRNA encoding those proteins, selected from the group consisting of pulmonary surfactant protein or a precursor thereof, CD44 and CD45 in the spherical cells formed. The spherical cells are cells that express one or more proteins, or mRNA encoding those proteins, selected from the group consisting of pulmonary surfactant protein, CD44 and CD45, but do not express one or more proteins, or mRNA encoding those proteins, selected from the group consisting of CD34 and CD90.

In the present invention, the spherical cells are cells that express pulmonary surfactant protein or a precursor thereof, or express mRNA encoding these proteins. Pulmonary surfactant is a substance that is secreted from type II alveolar epithelial cells and reduces alveolar surface tension, and is composed of 90% phospholipid and 10% protein. There are four known types of pulmonary surfactant proteins, consisting of types A to D, and these types have been disclosed by Kuroki et al. in "Surfactant Covering the Alveolar Surface - Structure, Function and Pathophysiology" (Proteins, Nucleic Acids and Enzymes, Vol. 43, No. 7, 1998, p. 834-846).

Although the spherical cells may be confirmed to be type II alveolar epithelial cell-like cells based on any of pulmonary surfactants A through D, pulmonary surfactant types B and C, or precursors thereof, can be used preferably. Confirmation is preferably based on pulmonary surfactant protein C (SP-C), or a precursor thereof, from the viewpoint that it is a pulmonary surfactant protein expressed in both type II alveolar epithelial cells and small airway epithelial cells. SP-C assists the main component of pulmonary surfactant in the form of phospholipid in reducing surface tension, and is also thought to contribute to recycling of surfactant in the alveolar space. ProSP-C protein is used particularly preferably for the purpose of detection in the present invention.

The amino acid sequence of mouse proSP-C protein and the nucleotide sequence of mRNA encoding mouse proSP-C protein are registered as GenBank Accession No. NP_035489 and Accession No. NM_011359.2, respectively.

In the present invention, the spherical cells express CD44 protein or mRNA encoding that protein. CD44 is an adhesion molecule that binds to components of the extracellular matrix such as hyaluronic acid, and in addition to being involved in cell-cell adhesion and cell-matrix adhesion, is also involved in morphogenesis, angiogenesis, infiltration and metastasis of cancer cells and lymphocyte homing.

Although CD44 protein has several splicing variants since CD44 mRNA is susceptible to selective splicing, expression may be confirmed for any of these splicing variants in the present invention. For example, the amino acid sequence of mouse CD44 protein and the nucleotide sequence of mRNA encoding mouse CD44 are registered as GenBank Accession No. CAA46883.1 and Accession No. BC005676.1, respectively.

In the present invention, the spherical cells express CD45 protein or mRNA encoding that protein. CD45 is a leukocyte common antigen (LCA), and is expressed in all hematopoietic-derived cells with the exception of mature erythrocytes and platelets.

Although CD45 protein has several splicing variants since CD45 mRNA is susceptible to selective splicing, expression may be confirmed for any of these splicing variants in the present invention. For example, the amino acid sequence of mouse CD45 protein and the nucleotide sequence of mRNA encoding mouse CD45 are registered as GenBank Accession No. AAA67166.1 and Accession No. M11934.1, respectively.

In the present invention, the expression level of CD34 protein, or mRNA encoding that protein, by the spherical cells is 5% or less of the expression level of all proteins or all mRNA, respectively. Namely, in the aforementioned preparation method, the spherical cells can be confirmed to be type II alveolar epithelial cell-like cells by confirming that the expression level of CD34 protein, or mRNA encoding that protein, in the formed spherical cells following step (3) is 5% or less of the expression level of all proteins or all mRNA, respectively.

CD34 is expressed in undifferentiated pluripotent stem cells or hematopoietic progenitor cells and the like. Although CD34 protein has several splicing variants since CD34 mRNA is susceptible to selective splicing, expression may be confirmed for any of these splicing variants in the present invention. For example, the amino acid sequence of mouse CD34 protein and the nucleotide sequence of mRNA encoding mouse CD34 are registered as GenBank Accession No. AAH06607.1 and Accession No. BC006607.1, respectively.

In the present invention, the expression level of CD90 protein, or mRNA encoding that protein, by the spherical cells is 5% or less of the expression level of all proteins or all mRNA, respectively. Namely, in the aforementioned preparation method, the spherical cells can be confirmed to be type II alveolar epithelial cell-like cells by confirming that the expression level of CD90 protein, or mRNA encoding that protein, in the formed spherical cells following step (3) is 5% or less of the expression level of all proteins or all mRNA, respectively.

CD90 is also referred to as Thy-1, and is expressed in thymocytes, CD34-positive precursor thymocytes, hematopoietic stem cells, nerves, human fetal liver cell subsets, umbilical cord blood cells and bone marrow cells.

Although CD90 protein has several splicing variants since CD90 mRNA is susceptible to selective splicing, expression may be confirmed for any of these splicing variants in the present invention. For example, the amino acid sequence of mouse CD90 protein and the nucleotide sequence of mRNA encoding mouse CD90 are registered as GenBank Accession No. NP_033408.1 and Accession No. BC054436.1, respectively.

In the present invention, pulmonary surfactant protein, CD44 protein, CD45 protein, CD34 protein and CD90 protein refer to that having amino acid sequence identity of 60% or more, 70% or more, 80% or more, 90% or more, 95% or more or 97% or more with the aforementioned mouse SP-C protein, mouse CD44 protein, mouse CD45 protein, mouse CD34 protein and mouse CD90 protein.

In addition, in the present invention, mRNA encoding pulmonary surfactant protein, CD44 protein, CD45 protein, CD34 protein and CD90 protein refer to that having nucleotide sequence identity of 60% or more, 70% or more, 80% or more, 90% or more, 95% or more or 97% or more with mRNA encoding the aforementioned mouse SP-C protein, mouse CD44 protein, mouse CD45 protein, mouse CD34 protein and mouse CD90 protein.

The percent identity between two amino acid sequences may be determined by visual inspection and mathematical calculation. Alternatively, the percent identity between two protein sequences may be determined through comparison of sequences using a GAP computer program available from the University of Wisconsin Genetics Computer Group (UWGCG) based on the algorithm by Needleman, S. B. and Wunsch, C. D. (J. Mol. Biol., 48: 443-453, 1970). Preferred default parameters of the GAP program include: (1) scoring matrix: blosum62 described in Henikoff, S, and Henikoff, J. G., (Proc. Natl. Acad. Sci. USA, 89: 10915-10919, 1992); (2) 12 gap weights; (3) 4 gap length weights; and (4) no penalty for terminal gaps.

Any other program used by persons skilled in the art may also be used for comparison of the sequences. The percent identity can be determined by, for example, comparison of sequences using a BLAST program described in Altschul et. al., (Nucl. Acids. Res., 25, pp. 3389-3402, 1997). This program is available from the websites of National Center for Biotechnology Information (NCBI) or DNA Data Bank of Japan (DDBJ) on the Internet. The conditions (parameters) for identity search by the BLAST program is described in detail on these sites. Although these parameters can be partly modified if necessary, search is generally carried out using the default values. Alternatively, the percent identity between two amino acid sequences may be determined using a program such as genetic information processing software GENETYX Ver. 7 (available from GENETYX CORPORATION) or FASTA algorithm, wherein search may be carried out using the default values.

The percent identity between two nucleotide sequences can be determined by visual inspection and mathematical calculation. Preferably, such comparison is carried out through comparison of sequences using a computer program. A particularly preferred computer program is a version 10.0 program "GAP", Wisconsin package of Genetics Computer Group (GCG, Madison, Wis.) (Devereux, et al, 1984, Nucl. Acids Res., 12: 387). The use of the "GAP" program enables comparison between two amino acid sequences and comparison between a nucleotide sequence and an amino acid sequence, in addition to comparison of two nucleotide sequences. The preferred default parameters for the "GAP" program include: (1) the GCG implementation of a unary comparison matrix (containing a value of 1 for identities and 0 for non-identities) for nucleotides, and the weighted amino acid comparison matrix of Gribskov and Burgess, Nucl. Acids Res., 14: 6745, 1986, as described in Schwartz and Dayhoff, eds., "Atlas of Polypeptide Sequence and Structure", National Biomedical Research Foundation, pp. 353-358, 1979, or other comparable comparison matrices; (2) a penalty of 30 for each gap for amino acids and an additional penalty of 1 for each symbol in each gap, or a penalty of 50 for each gap for nucleotide sequences and an additional penalty of 3 for each symbol in each gap; (3) no penalty for end gaps; and (4) no maximum penalty for long gaps. Other sequence comparison programs used by those skilled in the art can also be used. For example, the BLASTN program version 2.2.7, which is available via the National Library of Medicine website: http://www.ncbi.nlm.nih.gov/blast/bl2seq/bls.html, or the UW-BLAST 2.0 algorithm can be used. Setting of the standard default parameters for the UW-BLAST 2.0 is described at the following Internet site: http://blast.wustl.edu. In addition, the BLAST algorithm uses the BLOSUM62 amino acid scoring matrix, and optional parameters that can be used are as follows: (A) inclusion of a filter to mask segments of the query sequence having low compositional complexity (determined by the SEG program of Wootton and Federhen (Computers and Chemistry, 1993); also see Wootton and Federhen, 1996, "Analysis of compositionally biased regions in sequence databases", Methods Enzymol., 266: 544-71) or segments consisting of short-periodicity internal repeats (determined by the XNU program of Claverie and States (Computers and Chemistry, 1993)), and (B) a statistical significance threshold for reporting matches against database sequences or E-score (the expected probability of matches being found merely by chance, in accordance with the statistical model (Karlin and Altschul, 1990); if the statistical significance ascribed to a match is greater than the E-score threshold, the match will not be reported.); preferred E-score threshold values are 0.5, or in order of increasing preference, 0.25, 0.1, 0.05, 0.01, 0.001, 0.0001, 1e-5, 1e-10, 1e-15, 1e-20, 1e-25, 1e-30, 1e-40, 1e-50, 1e-75, or 1e-100.

The expression of the aforementioned proteins or mRNA encoding those proteins in the spherical cells can be confirmed using a known method. For example, expression of protein can be confirmed by ELISA, western blotting, radioimmunoassay or immunostaining using protein-specific antibody. Antibody commercially available from Abcam, for example, can be used for SP-C-specific antibody, CD44-specific antibody, CD45-specific antibody, CD34-specific antibody and CD90-specific antibody. At this time, antibodies to congenic markers such as CD45.1, CD45.2, CD90.1 or CD90.2 may also be used.

Expression of mRNA can be confirmed by detecting mRNA present within cells. Detection of mRNA can be carried out by RT-PCR, northern hybridization or in situ hybridization and the like. All of these methods are either commonly known and used methods for persons with ordinary skill in the art or can be carried out with reference to, for example, Molecular Cloning: A Laboratory Manual, Fourth Edition (Cold Spring Harbor Laboratory Press) or Current Protocols in Molecular Biology (John Wiley & Sons).

The percentage of spherical cells that express SP-C is preferably 80% or more and more preferably 90% or more.

Spherical cells derived from lung tissue obtained in the manner described above are referred to as lung mixed culture-derived epithelial cells (LMDEC).

In step (4) of the aforementioned method, the cell supernatant fluid is recovered by a method such as aspiration or decantation after the spherical cells have been formed.

Furthermore, the cell culture supernatant fluid can be recovered multiple times following step (4) by repeating step (3) and step (4) on the remaining cells multiple times as necessary. Although the number of times steps (3) and (4) are repeated after step (4) is carried out once can be suitably determined corresponding to conditions such as the animal species serving as the cell source, in the case of mouse-derived cells, for example, steps (3) and (4) can be repeated 2 to 5 times, while in the case of rat-derived cells, steps (3) and (4) can be repeated 2 to 12 times.

Although any method may be used to confirm the efficacy of the resulting cell culture supernatant fluid provided it is a method that is used in pharmacological experiments on therapeutic drugs for respiratory diseases, an example of a method that can be used is indicated below.

(1) The cell culture supernatant fluid is administered to a lung injury model animal either as is or after concentrating. Medium prior to culturing the cells is administered to the lung injury model animal as a negative control either as is or after concentrating.
   Although any model animal may be used for the lung injury model animal, an elastase-induced lung injury model mouse or rat or bleomycin-induced lung injury model mouse or rat, for example, can be used preferably.
   The animal housing period for animals administered negative control medium or cell culture supernatant fluid can be, for example, 3 weeks in the case of an elastase model or 2 weeks in the case of a bleomycin model.
(2) The animals are submitted for testing following administration and housing, and the lungs are removed to prepare tissue specimens.
   Lung tissue specimens obtained from the animals can be prepared in accordance with established methods. For example, the lung tissue can be fixed by immersing in formalin and the like followed by thinly slicing after embedding in paraffin or freezing, and then staining with hematoxylin-eosin stain.
(3) The mean linear intercept length is then measured in the tissue specimens. Mean linear intercept length can be measured by observing the prepared tissue specimens using a light microscope and the like.
(4) The mean linear intercept length in a sample from an animal administered the cell culture supernatant fluid is confirmed to have decreased significantly in comparison with the mean linear intercept length in a sample from an animal administered the negative control medium.

### B. Pharmaceutical Composition

The pharmaceutical composition of the present invention is able to effectively prevent or treat respiratory diseases as a result of containing a cell culture supernatant fluid obtained from lung tissue.

The cell culture supernatant fluid obtained from lung tissue can be used as is or concentrated and used as the pharmaceutical composition of the present invention. Although the culture supernatant fluid may be concentrated using any method, vacuum concentration, freeze concentration, membrane concentration or a combination thereof, for example, can be used preferably. Although the recovered culture supernatant fluid can be concentrated by about 2-fold to 100-fold, it may also be used after concentrating by about 2-fold to 20-fold or after concentrating by about 2-fold to 7-fold.

The amount of protein contained in the cell culture supernatant fluid can be, for example, 0.1 mg/ml to 200 mg/ml, and preferably 0.3 mg/ml to 100 mg/ml. This supernatant fluid can also be used as a solution from which serum protein and the like, for example, has been removed at the time of use. A concentrate from which water and serum protein components have been removed, or a reconstituted solution, obtained by adding media or solutions and the like following the removal thereof, can also be used as necessary.

In addition, a powder obtained by drying the cell culture supernatant fluid can also be used as is or as the pharmaceutical composition of the present invention by combining with other constituent components. Although any drying method may be used, freeze drying or spray drying is preferable.

There are no limitations on other constituent components able to be incorporated in the pharmaceutical composition of the present invention, and examples thereof include additional active ingredients, pharmaceutically acceptable carriers and combinations thereof.

Examples of additional active ingredients include, but are not limited to, steroids such as beclomethasone, fluticasone or budesonide, immunosuppressants such as FK506 or cyclosporin, antibiotics such as tobramycin or amikacin, antifibrotics such as pirfenidone, anticholinergics such as tiotropium, glycopyrronium or ipratropium, and β2-agonists such as salmeterol, indacaterol or formoterol. One of more types of these active ingredients may be incorporated in the pharmaceutical composition of the present invention corresponding to each of the target indications.

Examples of pharmaceutically acceptable carriers include, but are not limited to, buffers, excipients, diluents, stabilizers, antioxidants, preservatives, flavoring agents and combinations thereof that are not harmful to the subject of administration and do not impair the biological activity or properties of the active ingredient of the pharmaceutical composition of the present invention in the form of the cell culture supernatant fluid or additional active ingredients. Examples of buffers include phosphate buffer, citrate buffer and buffers containing other organic acids. Examples of excipients include calcium salts such as calcium carbonate or calcium phosphate, sugars such as sucrose, lactose, starch or cornstarch, cellulose derivatives such as hydroxypropyl cellulose or hydroxypropyl methyl cellulose, and gelatin. Examples of diluents include purified water or aqueous solutions such as physiological saline, alcohols such as ethanol, glycerol, propylene glycol or polyethylene glycol, and vegetable oils such as sesame oil, cottonseed oil or corn oil. Examples of stabilizers include chelating agents such as EDTA, amino acids such as glycine, glutamine, asparagine, histidine, arginine or lysine, monosaccharides such as glucose or mannose, disaccharides such as trehalose, and polysaccharides such as dextran, pectin, gua gum, xanthan gum or carrageenan. Examples of antioxidants include ascorbic acid, tocopherols, tocotrienols, carotenoids such as carotenes or xanthophylls, phenols such as flavonoids, amino acids such as methionine, and synthetic antioxidants such as tert-butylhydroquinone or butylated hydroxytoluene. Examples of preservatives include octadecyl dimethyl benzyl ammonium chloride, hexamethonium chloride, benzalkonium chloride, benzethonium chloride, phenol, butyl or benzyl alcohol, and p-hydroxyalkylbenzoates such as p-hydroxymethylbenzoate or p-hydroxyethylbenzoate. Examples of flavoring agents include sugar alcohols such as mannitol or sorbitol, sodium saccharin, aspartame, potassium acesulfame, thaumatin, stevia extract, 1-menthol, limonene and refined oils such as peppermint oil, lemon oil, orange oil, sage oil, rosemary oil, cinnamon oil, eucalyptus oil or clove oil.

There are no particular limitations on the form of the pharmaceutical composition of the present invention, and can be prepared in an arbitrary form in the manner of a solid form such as a powder, granules or tablets, a liquid form such as a solution, emulsion or dispersion, or a semi-liquid form such as a paste. Specific examples of drug forms include powders, granules, grains, tablets, pills, lozenges, capsules (including soft and hard capsules), chewable preparations and solutions.

Although the administration method of the pharmaceutical composition of the present invention can be suitably selected corresponding to such factors as the symptoms or age of the patient, administration is preferably in the form of transpulmonary, transnasal, oral or local administration. The dosage is such that a cell culture liquid, from which proteins of 3 kilodaltons or less and proteins of 30 kilodaltons or more have been removed and which has been concentrated 20-fold, is administered at 0.1 ml to 100 ml per 1 kg of body weight per administration, at 0.1 ml to 50 ml per 1 kg of body weight per administration, or at 0.5 ml to 5 ml per 1 kg of body weight per administration.

### C. Prevention or Treatment of Respiratory Diseases

"Respiratory diseases" targeted by the pharmaceutical composition of the present invention refer to diseases occurring in respiratory organs including the upper respiratory tract, trachea, bronchia and lungs. Examples of respiratory diseases include diseases of the trachea and bronchia, lung diseases and pleural diseases, according to the disease focus. In the present invention, preferable examples of respiratory diseases include, but are not limited to, lung diseases and bronchial asthma. In addition, examples of lung diseases include, but are not limited to, chronic obstructive pulmonary disease (COPD), pulmonary fibrosis, acute respiratory distress syndrome, pulmonary hypertension, pneumonia and pulmonary sarcoidosis.

The term "prevention" refers to partially or completely preventing the onset or progression of symptoms prior to the onset of symptoms of respiratory disease.

The term "treatment" refers to reversing, alleviating, reducing or inhibiting one or more symptoms of respiratory disease or partially or completely preventing the same.

Subjects able to be administered the pharmaceutical composition of the present invention are only required to be mammals, and examples thereof include humans, dogs, cats, cows, horses, domestic rabbits, hamsters, guinea pigs, pigs, monkeys, mice and rats.

The pharmaceutical composition of the present invention contains a cell culture supernatant fluid obtained from the lung tissue of a species that is the same as or different from the species targeted for administration. More specifically, the pharmaceutical composition of the present invention administered to a human contains cell culture supernatant fluid obtained from the lung tissue of one or more animals selected from the group consisting of humans, dogs, cats, cows, horses, domestic rabbits, hamsters, guinea pigs, pigs, monkeys, mouse and rats.

### D. Composition for Producing Pulmonary Surfactant Protein-Positive Cells

The composition of the present invention can also incorporate other constituent components in addition to the previously described lung tissue cell culture supernatant fluid or concentrate thereof. There are no particular limitations on the other constituent components able to be incorporated in the composition of the present invention, and examples thereof include additional active ingredients, pharmaceutically acceptable carriers and combinations thereof.

Examples of additional active ingredients include, but are not limited to, cell growth factors such as epithelial growth factor (EGF), insulin-like growth factor (IGF) or transforming growth factor (TGF), vitamins such as vitamin E, vitamin C or vitamin A, and minerals such as sodium, potassium, calcium or magnesium. In addition, examples of pharmaceutically acceptable carriers are the same as those used in the previously pharmaceutical composition.

There are no particular limitations on the form of the composition of the present invention, and can be prepared in an arbitrary form, in the manner of a solid such as powders, granules or tablets, a liquid form such as a solution, emulsion or dispersion, or a semi-liquid form such as a paste.

The amount of cell culture supernatant fluid incorporated in the composition of the present invention can be suitably set corresponding to the types of cells to be induced or the objective thereof. For example, the amount of cell culture supernatant fluid can be incorporated so that culture supernatant fluid, concentrate thereof or dried product thereof is added that contains 0.1 mg/ml to 200 mg/ml of protein per 10⁴ peripheral blood mononuclear cells.

### E. Method for Producing Pulmonary Surfactant Protein-Positive Cells

The present invention also relates to a method for producing pulmonary surfactant protein-positive cells that comprises a step for contacting the previously described lung tissue cell culture supernatant fluid or composition containing that culture supernatant fluid with cells such as peripheral blood mononuclear cells that are not destined to differentiate into pulmonary surfactant protein-positive cells. Examples of target cells not destined to differentiate into pulmonary surfactant protein-positive cells include, but are not limited to, peripheral blood mononuclear cells, mesenchymal stem cells and embryonic stem cells (ES cells).

There are no limitations on the method used to contact the culture supernatant fluid of the present invention with the target cells, and the target cells may be cultured in the culture supernatant fluid for the sake of convenience. The duration of culturing can be suitably adjusted by a person with ordinary skill in the art. In addition, a kit to be subsequently described may also be used to produce such pulmonary surfactant protein-positive cells.

### F. Kit for Producing Pulmonary Surfactant Protein-Positive Cells

The present invention also relates to a kit for producing pulmonary surfactant protein-positive cells that contains the previously described lung tissue cell culture supernatant fluid. The kit of the present invention can also include additional constituents in addition to the aforementioned lung tissue cell culture supernatant fluid, concentrate thereof or dried product thereof. Furthermore, a composition containing the aforementioned culture supernatant fluid may also be used.

Examples of other constituents able to be included in the kit of the present invention include, but are not limited to, cell culture medium, additional components for adding to the medium, an incubator for cell culturing and combinations thereof. In addition, an instruction manual describing the composition, components and use of the kit may also be included.

The medium for cell culturing may be any medium provided it is a medium that can be used to culture mammalian cells, and non-limiting examples thereof include serum-free medium such as PMPro, OptiPro or SFM, DMEM medium, EMEM medium, GMEM medium, MEM medium, RPMI medium, Ham F-21 medium, Eagle's MEM medium, RPMI1640 medium and mixed media thereof.

Examples of additional components for adding to the medium include, but are not limited to, serum, buffers, antibiotics, growth factors, cytokines, proteins or peptides, amino acids, sugars and culture supernatant concentrate. One or more types of these additional components may be included in the kit of the present invention corresponding to the purpose of use. In addition, a premixed mixture of these additional components with the medium may also be included in the kit of the present invention.

Examples of serum include bovine serum, fetal bovine serum and equine serum. Examples of buffers include HEPES buffer (2-[4-(2-hydroxyethyl)-1-piperazinyl] ethanesulfonic acid), phosphates such as potassium dihydrogen phosphate or sodium hydrogen phosphate, and mixtures of two or more types thereof. Examples of antibiotics include penicillin, streptomycin, amphotericin B and mixtures of two or more types thereof. Examples of growth factors include epithelial growth factor. Examples of cytokines include interleukin-1, interleukin-2, interleukin-3 and interleukin-4. Examples of proteins or peptides include serum albumin and L-alanyl-L-glutamine. Examples of amino acids include L-glutamine, L-alanine, L-asparagine, L-aspartic acid, L-glutamic acid, L-glycine, L-proline, L-serine, L-arginine, L-cysteine, L-histidine, L-isoleucine, L-leucine, L-lysine, L-methionine, L-phenylalanine, L-threonine, L-tryptophan, L-tyrosine, L-valine and mixtures of two or more types thereof. Examples of sugars include glucose.

The following provides a more detailed explanation of the present invention based on examples thereof. Furthermore, the present invention is not limited to these examples. A person with ordinary skill in the art would be able to easily modify or alter the present invention based on the description of the present description, and such modifications and alterations are included in the technical scope of the present invention.

### [Examples]

### Example 1: Preparation of Lung Mixed Culture-Derived Epithelial Cells (LMDEC)

### Materials and Methods

### <Animals>

Male and female C57BL/6J mice were purchased from CLEA Japan, Inc. (Tokyo, Japan).

### <Mixed culture and preparation of LMDEC>

Male mice (3-4 age in weeks) were anesthetized with pentobarbitalm, and intracardially perfused with 25 mL of ice-cold phosphate buffered saline (PBS) to thoroughly rinse lung blood cells. The lung lobe was disconnected from the trachea and the main bronchus was chopped into small pieces, and then digested in DMEM (Wako Pure Chemical Industries, Ltd., Tokyo, Japan) supplemented with 2 mg/mL type I collagenase (Worthington, Lakewood, NJ), 1 mg/mL Dispase (Life Technologies, Inc., Carlsbad, CA), 3 units/mL DNase (Qiagen, Valencia, CA), 0.1 mg/mL streptomycin, and 100 units/mL penicillin at 37 °C for 45 minutes with gentle shaking. The resulting small pieces were then sufficiently suspended to a single cell suspension, and the cells were passed through Cell Strainer (40 µm) (BD Biosciences, San Jose, CA), and then neutralized with FBS. Cells were washed with DMEM and resuspended in culture medium (DMEM supplemented with 10% FBS, 0.1 mg/mL streptomycin, 100 units/mL penicillin, and 2.5 µg/mL amphotericin B). Cells were seeded in culture dishes (cell density: 1.3 x 10⁶ cells/cm²) and incubated as "mixed culture" at 37 °C in a humidified atmosphere (5% CO₂). Two days after the culture, the culture dishes were washed several times with PBS to completely remove floating cells and then washed once with the culture medium. The cells were further incubated, and spherical cells floating or loosely adhering to adherent cells were collected as LMDEC by tapping the culture dishes on day 9 of culture.

### Result

From the above mixed culture, spherical cells were generated from spindle-shaped adherent cells, and the adherent cells at the bottom of the incubator became confluent on day 9 of culture, and aggregates of sphere-like cells were also observed on the adherent cells (Figure 1). The cell aggregates (apparent ratio: 2-3/1.5 mm²) were larger, more loosely adhered and flat, on day 7 of culture. Thereafter, the number of spherical cells floating or loosely adhered to adherent cells increased, and the adherent cells were covered on day 9 of culture. The yield of spherical cells was 2.4 ± 0.2 x 10⁶ cells/mouse on day 9 of culture.

### Example 2: Characterization of LMDEC

### Materials and Methods

### <Antibodies>

Rabbit anti-proSP-C antibody that recognizes an epitope at amino acid 11-27 in the N-terminus of mouse pro-SP-C was prepared by the Sigma-Aldrich Japan Genosys (Ishikari, Japan). Another rabbit anti-proSP-C antibody was purchased from Millipore (Billerica, MA). In addition, the following primary antibodies were used: anti-podoplanin/gp36 antibody (Abcam, Inc., Cambridge, UK); anti-CCSP/CC10 antibody (Santa Cruz Biotechnology, Inc., Santa Cruz, CA); PerCP-Cy (TM) 5.5-conjugated lineage antibody cocktail (anti-CD3ε, anti-CD11b, anti-CD45R/B220, anti-Ly-76, and anti-Ly-6G & 6C antibody (BD Biosciences); phycoerythrin (PE)-anti-CD34 antibody; biotin-, PE-, or Alexa Fluor (R) 647-anti-CD44 antibody; biotin-, Brilliant violet-, or PE-anti CD45.2 antibody; PE-anti-CD73 antibody; Alexa Fluor (R) 488-anti-CD 90.2 antibody; FITC- or PE-anti c-kit antibody; biotin- or PE-anti-Sca1 antibody (BioLegend Inc., San Diego, CA).

For fluorescence-conjugated primary antibody, fluorescent conjugate isotype controls corresponding to each primary antibody were used as negative controls in the flow cytometric analysis. In addition, the following secondary reagents and antibodies were used: Alexa Fluor (R) 350-streptavidin; Alexa Fluor (R) 594-Streptavidin; Alexa Fluor(R) 594-donkey anti-goat IgG; Alexa Fluor (R) 594-goat anti-hamster IgG; Alexa Fluor (R) 594-donkey anti-rat IgG; Alexa Fluor (R) 594-chicken anti-mouse IgG; Alexa Fluor (R) 350-goat anti-rabbit IgG; Alexa Fluor(R) 488-chicken anti-rabbit IgG (Life Technologies, Inc.); and DyLight (TM) 680-donkey anti-rabbit IgG (Rockland Immunochemicals Inc., Gilbertsville, PA).

### <Flow cytometric analysis>

Freshly recovered LMDEC and adherent cells in the mixed culture were sensitized with a 1:10 FcR blocking reagent for 15 minutes and subjected to cell surface staining for 15 minutes at 4 °C. After fixation and permeabilization (Fix/Perm buffer, BioLegend, Inc.), the cells were further subjected to intracellular staining for 15 minute at 4 °C. Primary antibodies and secondary reagents/antibodies were used at 1/100 and 1/200 dilutions, respectively. The cells thus obtained were filtered using Cell Strainer (70 µm) (BD Bioscience) and analyzed using a flow cytometer (FACSCanto (TM) II, BD Biosciences, Inc.). Positive staining cells were gated using appropriate isotype standards or negative control cells incubated with secondary reagents/antibodies. Data were collected and analyzed using FACSDiva (BD Biosciences, Inc.) and FlowJo 9.6.2 software (Tree Star, Inc., Ashland, OR).

### <RT-PCR>

Total RNA was prepared from LMDEC using RNAiso Plus (Takara Bio Inc., Seta, Japan) according to the manufacturer's instructions. Single-stranded cDNA was synthesized from the prepared RNA (3 µg) using Moloney murine leukemia virus reverse transcriptase (Life Technologies, Inc.) and using oligo (dT) primers (Life Technologies, Inc.) with a total volume of 20 µL. The obtained cDNA sample (1 µL) was subjected to PCR, and mouse SP-C or podoplanin/T1α gene was amplified using specific primers (for SP-C, 5'-TGGACATGAGTAGCAAAGAG-3' as sense primer and 5'-GTAGCAGTAGGTTCCTGGAG-3' as antisense primer; for T1α, 5'-GATCACAGAGAACACGAGAG-3' as sense primer and 5'-TCTTTCCTTTGGTACTGCTG-3' as antisense primer). Mouse glyceraldehyde-3-phosphate dehydrogenase (GAPDH) cDNA as an internal standard was amplified using specific primers (5'-GACCACAGTCCATGACATCACT-3' as a sense primer and 5'-TCCACCACCCTGTTGCTGTAG-3' as an antisense primer). The thermal cycler was set as follows: for SP-C and T1α, 35 cycles of 98 °C for 30 seconds, 56 °C for 30 seconds, 68 °C for 7 minutes, and 68 °C for 7 minutes; for GAPDH, 26 cycles of 98 °C for 30 seconds, 60 °C for 30 seconds, and 68 °C for 30 seconds. The amplified products were separated in a 1.2% agarose gel and visualized under UV irradiation by ethidium bromide staining. Specific amplifications of mouse SP-C (365 bp), T1α (414 bp), and GAPDH (450 bp) were observed.

### <Immunofluorescence observation>

The LMDEC obtained from the mouse was dispersed in the same culture medium as in Example 1, and seeded on an 8-well chamber slide (Thermo Inc., Waltham, MA). On Day 0, within 3 hours after seeding and after the time indicated in the figure (however, if it exceeds 3 days, half of the culture medium was replaced with fresh medium for every 3 days and maintained), the cells are washed with ice-cold PBS and fixed with 4% PFA/0.1 M NaPB. Then, the cells were permeabilized for 3 minutes with 0.1% Triton X-100/PBS and washed with ice-cold PBS. After treatment with SuperBlock (Thermo, Inc.) in PBS for 15 minutes, the cells were incubated with combination of a primary antibody (anti-proSP-C antibody and/or anti-M2 flag antibody) and a secondary antibody (Alexa Fluor(R) 488-chicken anti-rabbit IgG antibody and/or Alexa Fluor (R) 594-goat anti-mouse IgG antibody (Life Technologies, Inc.)), and observed under a fluorescence microscope. The nuclei were stained using 4',6-diamino-2-phenylindole (DAPI) (Dojindo Laboratories, Kumamoto, Japan).

In the Sca1 positive cell depletion test, mixed cultures without Sca1 cells were prepared using anti-Sca 1µ bead kit (Miltenyi Biotech, Inc.). Then, LMDEC obtained from normal mixed cultures or mixed cultures without Sca1 cells was subjected to double staining with anti-proSP-C antibody and biotin-anti-CD45.2 antibody in combination with secondary antibodies (Alexa Fluor (R) 488-chicken anti-rabbit IgG antibody and Alexa Fluor (R) 594-streptavidin (Life Technologies Inc.)).

### <Proliferation assay>

Ten µM 5-ethynyl-2'-deoxyuridine (EdU) was loaded for 6 hours on adherent cells in the mixed cultures after collecting LMDEC, and the cells were washed with PBS and then fixed with 4% PFA/0.1 M NaPB. EdU incorporated into the DNA was detected with Alexa Fluor (R) 488- or 647-azide according to the attached protocol of Click-iT (R) EdU imaging kit (Life Technologies, Inc.). The cells with fluorescein-labeled nuclei were subjected to fluorescent immunostaining using the antibodies listed in Table 1 and examined using FACSCanto II.

### <Statistical analysis>

Data are presented as mean ± standard error. Statistical analysis was performed using GraphPad Prism Version 6 (GraphPad Software Incorporated, Inc., San Diego, CA). Statistically significant differences were detected by Student's t test or Student's t test following dispersion analysis, and p value < 0.05 was considered significant.

### Result

### <Flow cytometric analysis>

As a result of flow cytometric analysis, most of LMDEC (96.4% ± 1.1%) was SP-C positive (FIG. 2A).

Up to now, it is known that CD44 is constitutively expressed in at least *in vitro* culture in cells of the alveolar epithelial cell lineage, i.e., cells that are destined to differentiate into alveolar epithelial cells (Am J Physiol Lung Cell Mol Physiol, 2009, 296, L442-452). Expression of CD44 on LMDEC was investigated and it was found that LMDEC exceeding 90% (FIG. 2A) was CD44 positive.

On the other hand, CD45, a hematopoietic cell marker, is generally known not to be expressed in type II alveolar epithelial cells (Am J Physiol Lung Cell Mol Physiol, 2009, 296, L442-452). Surprisingly, nearly 90% of LMDEC was positive for CD45 (91.1 ± 3.1%) (FIG. 2A). Furthermore, the main population of LMDEC (LMDEC^{Maj}) co-expressed SP-C, CD44 and CD45 (FIG. 2B).

To clarify whether LMDEC contains other cell populations of pulmonary stem cells/progenitor cells, we examined using the following various markers: CD34 (a hematopoietic stem cell/progenitor cell marker that covers from earlier pluripotent progenitors to progenitor cells restricted by late cell lineage (Proc Natl Acad Sci U.S.A., 2009, 106, 8278-8283), and this is a concentration marker tool for bronchoalveolar epithelial cells (BASC)); CD90 (one of main markers of mesenchymal stem cells (MSC) present in tissues (Lab Invest, 2011, 91, 363-378)). In LMDEC, little expression of CD34 and CD90 was observed (FIG. 2A). On the other hand, small cell populations showing SP-C positive/CCSP positive/Sca 1 positive were observed in LMDEC. This suggests that LMDEC contains bronchoalveolar epithelial cells (BASC) (FIG. 2C). BASC has the ability to differentiate into both respiratory tract and alveolar lineage (Cell, 2005, 121, 823-835). This suggests that LMDEC^{Maj} may be partly derived from BASC.

Next, lung mixed cultures with or without Sca1 positive cells were prepared, and recovery rates of SP-C positive/CD45 positive LMDEC were examined under each condition. Depleting Sca1 positive cells from the mixed cultures did not affect the recovery of SP-C positive/CD45 positive LMDEC (FIG. 2C). This suggests that BASC may not differentiate into LMDEC^{Maj} at least in the culture conditions used this time.

### <RT-PCR>

Regarding LMDEC, we investigated time-dependent changes in mRNAs of SP-C and podoplanin/T1α which is a marker of type I alveolar epithelial cells. A decrease in SP-C mRNA expression was observed, and an increase in T1α mRNA expression was observed in inverse proportion thereto (FIG. 3A). When subjected to immunofluorescent staining using anti-proSP-C antibody and anti-podoplanin antibody (anti-gp36 antibody), LMDEC immediately after collection showed a positive immune response to pro-SP-C (in cells of more than 90% of the total cells), and when cultured for 7 days, gp36 became positive (in cells of more than 80% of the total cells) (FIG. 3B). When the change in this marker expression was observed over time, an inverse correlation was confirmed according to the analysis result of mRNA (FIG. 3C). These results suggest that LMDEC can differentiate into type I alveolar epithelial cells.

### <Characterization of adherent cells in mixed culture>

As shown in FIG. 1, LMDEC began to increase markedly as floating or loosely attached cells 7 days after culture. In order to evaluate the origin and self-renewal ability of LMDEC^{Maj} recognized as SP-C positive/CD44 positive/CD45 positive, the cell population of adherent cells in mixed culture was focused on stem cells and examined. LMDEC was washed away on day 7 of culture and the remaining adherent cells were labeled with EdU, recovered, subjected to fluorescent immunostaining and EdU detection reaction, and analyzed by flow cytometry. As shown in Table 1, Lin negative/Sca1 positive/c-kit positive LSK cells (primitive hematopoietic stem cells), and the CD45 negative/CD90 positive/CD 73 positive MSCs were included in the adherent cells at the rates of 1.4% and 4.7%, respectively. The percentages of EdU labeled cells in LSK and MSC were 76% and 11.3%, respectively. BASC was 6.6% of total adherent cells, 44.4% of which were EdU positive.

**[Table 1]**

| Stem cells/progenitor cells and LMDEC^{Maj} cells in adherent cells of mixed culture | | | |
|---|---|---|---|
| Name | Profile | Contents (% in total cells) | Edu positive |
| BASC | SP-C⁺/CCSP⁺/Sca1⁺ | 6.6 ± 0.7 | 44.4% |
| LSK (HSC) | Lin⁻/Sca1⁺/c-kit⁺ | 1.4 ± 0.1 | 76.0% |
| MSC | CD45⁻/CD90⁺/CD73⁺ | 4.7 ± 0.2 | 11.3% |
| LMDEC^{Maj} | SP-C⁺/CD44⁺/CD45⁺ | 17.5 ± 0.9 | 36.8% |
| | | | (n=4-6) |

These results suggest that different types of stem cells arising from internal or external origin are contained in adherent cells of mixed cultures. Notably, SP-C positive/CD44 positive/CD45 positive LMDEC^{Maj} was also contained as 17.5% of total adherent cells, 36.8% of which were EdU positive and contained in the adherent cells of the mixed cultures.

### Example 3: Administration of supernatant fluid of mixed culture of lung tissue-derived cells to pulmonary injury model mice

### <Preparation of supernatant fluid of mixed culture of lung tissue-derived cells>

Mixed culture of lung tissue-derived cells was performed according to the method of Example 1. The culture supernatant fluid was collected and centrifuged at 1,500 rpm for 5 min, and then the supernatant fluid was recovered and filtered through a 0.22 µm filter. The filtrate was applied to a centrifuge tube with an ultrafiltration filter, and a fraction containing protein having a molecular weight of 3-50 KDa was concentrated and purified to prepare a solution (concentration ratio: 25 times). This solution was used as supernatant fluid of mixed culture of lung tissue-derived cells in experiments.

### <Administration of supernatant fluid of mixed culture of lung tissue-derived cells to pulmonary injury model mice>

Elastase (30 µg/mouse) was transtracheally administered as a single dose (day 0) to mice. Thereafter, the mice were divided into three groups of (1) to (3), and each group was transtracheally administered 75 µl of each of (1) PBS, (2) control medium (10% FBS/DMEM), or (3) supernatant fluid of mixed culture of lung tissue-derived cells, on 3 day, 7 day, 10 day, 14 day and 17 day (total 5 times). Thereafter, the mouse was tested, the lungs were removed, fixed and sectioned, then stained with hematoxylin and eosin, and the mean linear intercept length was measured. By comparing inhibitory effects on the increase in mean alveolar wall distance induced by elastase for each administration group, the effect on emphysema formation was compared and examined.

### <Result>

The results are shown in Fig. 4.

Only the supernatant fluid of mixed culture of lung tissue-derived cells showed inhibitory effect on the increase in mean alveolar wall distance induced by elastase (emphysema formation in the lung).

### Example 4: Induction of cells using LMDEC culture supernatant fluid

(Part 1) Cell induction using mouse LMDEC culture supernatant fluid
   (1) Mouse LMDEC is prepared from mouse fresh lung according to a standard method.
   (2) Blood is drawn from the mouse and mouse mononuclear cells are removed using a vacutainer.
   (3) Using the aforementioned mouse mononuclear cell as a raw material, CD45 positive cells are isolated using MACS.
   (4) Cells are seeded in 8 well chambers so as to be 4 x 10⁵.
   (5) Culture supernatant is isolated from the mouse LMDEC prepared in (1), and cells and cell debris, etc., are removed using Millipore filter (5 µm) to obtain LMDEC culture supernatant fluid. The supernatant fluid is isolated twice, as Supernatant fluid 1 and Supernatant fluid 2, respectively.
   (6) The medium is slowly removed from each chamber of (4), and 200 µl of the supernatant fluid prepared in (5) is added.
   (7) After culturing (6) for 4 days, the medium is removed, and cells are washed with PBS and fixed with formalin. After immunostaining with SPC and gp36 as primary antibodies, fluorescence microscope observation is performed. The results are shown in Figure 5. SPC is shown in green (arrowhead), while gp36 is shown in red (arrowhead). Induction was also observed at the gene level.
(Part 2) Similar results were obtained when human monocytes were used instead of mouse monocytes.

## Claims

1. A culture supernatant fluid of cells derived from lung tissue.

2. The culture supernatant fluid according to claim 1, wherein the cells derived from lung tissue are spherical cells expressing one or more proteins, or mRNA encoding the same, selected from the group consisting of pulmonary surfactant protein or a precursor thereof, CD44 and CD45.

3. The culture supernatant fluid according to claim 2, wherein the pulmonary surfactant protein is pulmonary surfactant protein C (SP-C).

4. The pharmaceutical composition according to claim 2 or 3, wherein the expression level of one or more of the proteins, or mRNA encoding the same, selected from the group consisting of CD34 and CD90 is 5% or less of the expression level of all proteins or all mRNA.

5. A method for preparing a culture supernatant fluid of cells derived from lung tissue, comprising:
(1) a step for forming a cell suspension by suspending cells isolated from lung tissue in a medium,
(2) a step for culturing the cell suspension followed by removing non-adherent cells,
(3) a step for further culturing the remaining cells in a medium to form spherical cells, and
(4) a step for recovering the cell culture supernatant fluid.

6. A pharmaceutical composition for the treatment and/or prevention of respiratory diseases, containing the culture supernatant fluid according to any one of claims 1 to 4, or the culture supernatant fluid prepared by the method according to claim 5.

7. The pharmaceutical composition according to 6, wherein the respiratory disease is a lung disease or bronchial asthma.

8. The pharmaceutical composition according to claim 7, wherein the lung disease is selected from the group consisting of chronic obstructive pulmonary disease (COPD), pulmonary fibrosis, acute respiratory distress syndrome, pulmonary hypertension, pneumonia and pulmonary sarcoidosis.

9. A method for producing pulmonary surfactant protein-positive cells, comprising: contacting the culture supernatant fluid according to any one of claims 1 to 4, or a culture supernatant fluid prepared by the method according to claim 5 with cells selected from the group consisting of peripheral blood mononuclear cells, mesenchymal stem cells and embryonic stem cells (ES cells).

10. A kit for producing pulmonary surfactant protein-positive cells, including the culture supernatant fluid according to any one of claims 1 to 4 or a culture supernatant fluid prepared by the method according to claim 5.


SEQUENCE LISTING

<110>   Ube Industries, Ltd.
        Chiba University

<120>   Cell culture supernatant derived from lung tissue

<130>   P150698

<150>  JP2014-259827
<151>  2014-12-24

<150>  JP2014-259829
<151>  2014-12-24

<160>   6

<170>   PatentIn version 3.5

<210>   1
<211>   20
<212>   DNA
<213>   Artificial Sequence

<220>
<221>   primer

<400>   1
tggacatgag tagcaaagag                                                   20


<210>   2
<211>   20
<212>   DNA
<213>   Artificial Sequence

<220>
<221>   primer

<400>   2
gtagcagtag gttcctggag                                                   20


<210>   3
<211>   20
<212>   DNA
<213>   Artificial Sequence

<220>
<221>   primer

<400>   3
gatcacagag aacacgagag                                                   20


<210>   4
<211>   20
<212>   DNA
<213>   Artificial Sequence

<220>
<221>   primer

<400>   4
tctttccttt ggtactgctg                                                    20


<210>   5
<211>   22
<212>   DNA
<213>   Artificial Sequence

<220>
<221>   primer

<400>   5
gaccacagtc catgacatca ct                                                 22


<210>   6
<211>   21
<212>   DNA
<213>   Artificial Sequence

<220>
<221>   primer

<400>   6
tccaccaccc tgttgctgta g                                                  21